# EUROPEAN PATENT APPLICATION

(11) **EP 1 837 658 A1**
(43) Date of publication of application: **26.09.2007**
(21) Application number: 06005856.7
(22) Date of filing: 22.03.2006
(51) Int. Cl.: G01N 33/68

(54) **Cytokine-based pyrogen test**

(71) Applicant: Paul-Ehrlich-Institut Bundesamt für Sera und Impfstoffe, 63225 Langen (DE)
(72) Inventor: Montag-Lessing, Thomas, 64283 Darmstadt (DE); Spreitzer, Ingo, 65203 Wiesbaden (DE); Löschner, Bettina, 63303 Dreieich-Sprendlingen (DE)
(74) Representative: Krauss, Jan

(57) **Abstract**

The invention relates to a method for detecting the presence of a pyrogen in a sample comprising the steps of subjecting said sample to a solution comprising monocytes, incubating the sample and the solution, detecting in one reaction vessel the presence of at least two endogenous pyrogens. The invention further pertains to the use of the inventive method for evaluating the safety or possible contamination of a product for use with humans or animals. Further the invention relates to a kit for performing the inventive method, wherein the kit comprises two or more anti-endogenous-pyrogen-antibodies for detecting a pyrogen or at least two nucleic acid probes or primers for detecting an mRNA of a gene encoding an endogenous pyrogen. The invention further relates in particular to an ELISA kit, more in particular to an ELISA kit comprising an anti-IL-6- antibody, anti-IL-1β-antibody, anti-TNF-α-antibody, anti-prostaglandine E2(PGE2)-antibody and/or anti-Neopterine-antibody.

## Description

### Introduction

### Significance of pyrogens

Pyrogens as fever-inducing substances have been studied extensively over the past 50 years. The concept of fever has been known for a much longer time; as early as 100 years A/D it was described as a therapeutic remedy by Ruphos of Ephesos. The idea of antipyretic treatments is more recent, and the idea of the therapeutic value of fever has been abandoned. The association of fever with intravenous injections dates from as early as 1642 (Westphal, 1977). In the 18^{th} century, the incidence of fever was attributed to the intravenous injection of putrid material into animals (van Haller, 1757-1766). Hort and Penfeld developed the first rabbit pyrogen test in 1912 and demonstrated that Gram-negative bacteria were pyrogenic whereas Gram-positive bacteria were not. Furthermore, they deduced that the pyrogenic principle was probably a heat-stable bacterial substance. The purified endotoxin principle was first introduced by Boivin and Mesrobeanu in 1932 with a trichloroacetic acid extraction procedure. Since then, more highly purified preparations have been made available (Westphal et al., 1952). During the second world war, the significance of fever-inducing substances in large volume parenterals such as infusion therapeutics was recognized and extensive research as to the pyrogenic principle and the detection and elimination of pyrogens from intravenously applied drugs has been done ever since.

### Pyrogens

Pyrogens have been divided into two classes: exogenous pyrogens such as endotoxin from Gram-negative bacteria that induce fever when applied intravenously, and endogenous pyrogens that are induced inside the body as a reaction to the contact with exogenous pyrogens and cause an elevation in body temperature. The cells that produce the endogenous pyrogens are mainly the monocytes, a subfraction of the white blood cells. In the body, the endotoxin or lipopolysaccharide binds to a specific receptor complex on the monocyte, the CD 14 receptor, under involvement of the so-called toll-like receptor 4. This causes a cascade of reactions leading to the induction of endogenous pyrogens such as Interleukin-1β (IL-1β), Interleukin-6 (IL-6) and Tumor Necrosis Factor-α (TNF-α). The Interleukin-8 (IL-8) is also induced in the monocyte by exogenous pyrogens, but is a chemokine by nature with different functions than the other cytokines. The endogenous pyrogens then cause a change in the thermoregulation in the brain towards a higher body temperature. Known pyrogenic substances are endotoxins (LPS, lipopolysaccharide) as components of the cell walls of Gram-negative bacteria, for example the LPS from *Escherichia coli* 0113: H10, LTA (lipoteichoic acid) as a cell wall component of Gram-positive bacteria, *e.g.* from *Staphylococus aureus* (Morath *et al.,* 2002), furthermore peptidoglycan, which are common to both Gram-negative and Gram-positive bacteria, making up 40-90% of the cell wall's dry weight, and the mycoplasmal macrophage-activated lipopeptide (MALP). Other pyrogenic substances comprise bacterial exotoxins, *e.g.* from *Streptococcus,* enterotoxins, *e.g.* from *Staphylococcus aureus* (both belonging to the so called superantigens), and components of fungi. All of them cause fever when injected.

All endotoxins have a common general structure. Basically, three regions are distinguishable. The Lipid A portion is covalently attached to the core region which can be divided into an inner and an outer core. The inner core contains a high proportion of sugars that occur in LPS only, for example the 2-keto-3-deoxy-D-manno-octulosonic acid (KDO). To the outer core, there is usually attached a polymer of repeating saccharide units called the O-polysaccharide or O-chain (O-antigen), typically composed of common hexoses, as is the outer core.

The endless variety of serotypes is a consequence of the almost limitless variability of the O-chain structure (differences in sugars, sequence, chemical linkage, substituents and ring forms). The O-chain is also the part expressed on the surface of the bacterium and is therefore the major target for the host antibody response. The structures in the core region are more limited, whereas the inner core is more conserved than the outer one (identical for example for all *Salmonella* species).

In 1960, it became clear that the lipid portion contained the pyrogenic activity as mutants lacking parts of the O-chain were found to retain biological activity (Tanamoto *et al.,* 1984).

Later, it was found that the biological activity of the Lipid A is determined by the acylation pattern (Rietschel *et al.,* 1993), with up to 7 acyl substituents, the length of those acyl substituents and the phosphorylation state of the disaccharide backbone (Takeda *et al.,* 1992).

In contrast, the pyrogen LTA binds to the toll-like receptor 2 (Takeuchi *et al.* 1999) causing a similar cascade and causing fever along the same lines as LPS. For the MALP, an activation of the toll-like receptors 2 and 6 is discussed (Deiters *et al.,* 2003). The structure of LTA from *S. aureus* comprises a phosphoglycerol backbone esterified with D-alanine (70%) and D-N-acetylglucosamine (15%). Deliberate hydrolysis of the D-alanine substituents revealed a crucial role of those for the biological activity of the LTA, in this case from *Staphylococcus aureus* (Morath *et al., 2001).*

The need for pyrogens standards was first discussed in the 1950s at a meeting of the WHO expert group. The first ones were preparations from *Proteus vulgaris* and *Serratia marcescens* in 1953, both were evaluated for their potency in the rabbit pyrogen test. *Serratia* turned out to be more potent but was not chosen to be an international pyrogen standard. By 1956, the committee had selected an LPS preparation from *Shigella dysenteriae* to be the first world's pyrogen standard. Later it turned out to be inferior to other standards such as LPS from *Salmonella abortus equi* (Novo Pyrexal) or *E. coli* 055 (EC-2). *Escherichia coli* is a Gram-negative bacterium belonging to the normal gut flora of humans. Furthermore, it is commonly associated with intestinal and urinary tract infections. However, the LPS from *E. coli* alone can be the cause of a number of diseases. Being a gut commensal as mentioned above, the human intestine contains many hundred grams of the bacterium. However, in case of a breakdown of the intestinal barrier, as is the case e.g. in shock or other severe illness, the resulting systemic inflammation can lead to multiple organ failure, disseminated intravascular coagulation (DIC) and death.

*E. coli* LPS is highly toxic, *Salmonella species* LPS is typically considered to be as active as *E. coli* LPS. *Serratia marcencens* was long thought to be non-pathogenic. In 1951/2 the US army even conducted an operation called "Sea-Spray" to study wind currents. They burst balloons with *Serratia marcescens* over San Francisco. Later, *Serratia* was associated with pulmonary and urinary tract infections, as well as endocarditis, osteomyelitis, septicemia, wound and eye infections and meningitis. *Bordetella pertussis,* the agent of the whooping cough, possesses a so-called LOS (lipooligosaccharide), i.e. the bacterium only expresses a short non-repeating polysaccharide unit. Since the Lipid A expresses only five acyl chains, a low activity can be expected and has been shown. *Staphylococcus aureus* is a clump-forming Gram-positive coccus that, besides being a major cause of nosocomial and wound infections, can cause food poisoning (enterotoxins, belonging to the superantigens) and Toxic Shock Syndrome (TSS) by release of Toxic Shock Syndrom Toxin (TSST) and, addionally, superantigens. Interestingly, the first description of a superantigen concerned the old known erythrogenic toxin (scarlet fever toxin) of *Streptococcus pyogenes* which had been called in the respective publication "pyrogenic exotoxin".

### Mechanisms of pyrogenic effects

Some two decades ago the association of LPS with host cells resulting in their activation was believed to be based on an interdigitation of the lipid A acyl groups into the membrane of cellular targets (Kabir *et al.,* 1978). As a result of the work of Morrison, Wright, Ulevitch, Tobias and others specific biding proteins and receptors are now known to be involved in the recognition of LPS by the host cell.

The Cluster of Differentiation (CD) 14 receptor, a glycoprotein whose significance was re cognized in 1990, exists in a membrane-bound (mCD14, a 53 kDa protein) and a soluble form. The former is embedded in the plasma membrane via a GPI anchor. The latter, sCD14, is capable of inhibiting LPS activity (Schütt *et al.,* 1992), but additionally LPS/sCD14 complexes can induce biological responses in certain CD-14-negative cells such as endothelium (Frey *et al.* 1992). Its concentration in normal serum is approximately 14-22 µg/ml but can reach a maximum of 200 µg/ml in acute-phase serum. The LBP is capable to transfer LPS to the cellular binding site, the CD14 protein. Furthermore, kinetic experiments showed that upon longer exposure to LPS, the LBP mediates diffusion of LPS into HDL, thus neutralizing the endotoxin.

The BPI is a protein of the innate immune system that is only cytotoxic for Gram-negative bacteria and found in polymorphonuclear leucocytes (PMNs). The BPI is highly conserved (> 60% homology) between species and inhibits the growth of a wide range of Gram-negative bacteria without any toxicity for Gram-positive bacteria or eukaryotic cells. Upon binding of BPI to the outer membrane, several immediate but reversible effects are manifest, including growth arrest, membrane permeability changes and activation of enzymes degrading phospholipids and peptidoglycans.

### Receptors

The innate immune system of mammals uses the family of toll-like receptors (TLR) to engage microorganisms by recognizing so-called PAMPS (pathogen-associated molecular patterns). The term TOLL originally referred to a cell surface receptor governing dorsal/ventral orientation in the early Drosophila larvae. So far, more than 10 human members of this receptor family have been identified and recent years have seen rapid progress in identifying the roles of these receptors. A homologue of the Drosophila Toll receptor was identified in mammals. Toll antigen-presenting cells such as macrophages express TLR on their surface, bind PAMP, and initiate a pathway that induces host defense through reactive oxygen and nitrogen intermediates. It also initiates adaptive immunity by inducing pro-inflammatory cytokines. All TLRs activate a common transduction pathway that culminates in the activation of different factors: the activation of NFκB (nuclear factor-κB), the mitogen-activated protein kinases (MAPKs), ERK (extracellular signal-regulated kinase), p38 and the c-Jun N-terminal kinase (JNK). For binding, the TLR4 receptor dimerizes and the intracellular cascade starts.

LPS binds to LBP and this complex is subsequently recognized by CD14, as mentioned above a glycosylphosphatidylinositol-anchored molecule expressed on monocytes/macrophages and neutrophils. It seems likely today that CD14 catalyzes the insertion of the LPS molecules into either the plasma membrane or directly into the receptor complex which consists of the TLR4 receptor and an extracellular protein called MD-2. After binding, the LPS is rapidly delivered into the cytoplasma of the target cell. Thereupon, NFκB is activated.

### Intracellular signaling

Upon stimulation, MyD88 recruits serine/threonine kinases of the IRAK (IL-1 receptor associated kinase) family. These then act through TRAF-6 (TNF-receptor associated factor) to promote both MAP kinase cascades and the NFκB-inducing cascades. In the latter, TRAF-6 activates two kinases (Ikα and β) to form a dimer (IkK) that phosphorylates an inhibitory protein, IKB. The latter inhibits NFκB. Upon phosphorylation, the IkB dissociates, is degraded and the NFκB enters the nucleus. The transcription of pro-inflammatory cytokines is initiated.

### Cytokines

### IL-1β

The production of IL-1β has been demonstrated in nearly all tissues containing mononuclear phagocytes, including blood (monocytes), lung (alveolar macrophages), liver (Kupffer cells), bone marrow, spleen, and umbilical cord/placenta. The IL-1β exists in the mononuclear cells as a preformed molecule, the pro-IL-1β. Upon stimulation, a protease is activated, the IL-1 Converting Enzyme (ICE), whose catalytic function is essential for the generation of mature, extracellular IL-1β.

The final role of IL-1β in the generation of fever has yet to be determined. The most direct evidence of its involvement comes from studies where recombinant IL-1ra was injected. Whereas injection of Il-1 induces fever, fever reaction after administration of LPS was significantly attenuated as well as the IL-6 increase in plasma levels which normally parallels fever.

Anyway, substantial evidence exists that cytokine action is pivotal to the generation of fever (Rothwell and Hopkins 1995). Direct injection of IL-1β into the brain induces increases in body temperature (Rothwell *et al.,* 1990). Studies have demonstrated an inhibition of the increase of hyperthalamic IL-6 after application of neutralizing antibody to IL-1β suggesting a secondary role of IL-6 (Klir *et al.,* 1994). The importance of brain IL-6 in fever was further demonstrated by Chai *et al.* 1996.

### IL-6

TM IL-6 belongs to the IL-6 cytokine superfamily, it also causes fever. All members of the family employ the gp130 receptor subunit for signalling. Binding of a ligand to this receptor results in homodimerization and activation of receptor-associated kinases, the Janus-kinases (JAKs). These JAKs in turn phosphorylate cytosolic proteins called signal transducers and activators of transcription (STATs), causing them to dimerize through interaction of their respective Src homology 2 (SH2) domains. The IL-6 family, for instance, activates STAT 3 via the JAKs 1, 2 and TYK 2.

### TNF-α

TNF-α acts as a homotrimer on 4 receptors, two of which are involved in the inflammatory and acute phase response of the organism, it also causes fever.

### Effects of cytokines

The pro-inflammatory cytokines IL-1β, IL-6 and TNF-α are the most important fever inducing cytokines. They have many overlapping functions referred to as redundancy. Therefore, many partially contradicting publications can be found as to which one plays the key role in the pathological effects in the organism. Thus, so far it has not been straight forward to use these for pyrogen testing.

### Pyrogen testing with rabbits

The rabbit pyrogen test has been the gold standard in pyrogen testing since 1942, when it was introduced into the USP (United States Pharmacopoeia). The rabbit species was chosen by Seibert, who also discovered the pyrogenic principle (Seibert, 1925). It involves a measurement in body temperature after the application of not more than 10 ml/kg bodyweight of the substance to be tested. The rabbit has a labile thermoregulation and tends to give false-positive results. This is one of the drawbacks of the rabbit test. Also, the very rigid fixation and the handling (injection procedure) can cause a hyperthermia due to excitement. This is a further drawback of the rabbit test. In fact, it has been reported that the fixation and lack of movement can cause hyperthermia yielding false-negative results.

In its simplest form, the test involves measuring a rise in body temperature following intravenous injection of a test solution. Temperature is to be measured by a clinical thermometer or a probe inserted into the rectum of the rabbit at a depth of not less than 7.5 cm. The test is limited to substances that can be injected in doses of not more than 10 ml/kg body weight within a period of not more than minutes. Recently, a study involving 171 rabbits performed at the Paul-Ehrlich-Institute (PEI) indicated that the *in vivo* fever threshold of the most sensitive rabbits is at 50 pg/ml (0.5 Endotoxin Units/ml) of the international WHO standard from *E. coli* 0113:H10 (Hoffmann *et al.,* 2005). A challenge of 5 EU/kg applied in 10 ml/kg (corresponding to 0.5 EU/ml) intravenously evoked a fever reaction in 50% of the rabbits. All rabbits reacted with temperature increase when challenged with 20 EU/kg. This fever threshold of 0.5 EU/ml was chosen as the concentration that has to be found by the human whole blood assay, which would be required to replace the *in vivo* rabbit test.

### LAL (limuls amoebocyte Lysate Test)

When in contact with the lipid A portion of endotoxin, the amoebocytes from Limulus polyphemus (horseshoe crab) coagulate due to an enzymatic reaction (Levin and Bank, 1964). In the presence of calcium, the clotting enzyme as zymogen is activated by a serin protease and coagulates the clottable protein in the lysate, producing a smaller clot protein. The clotting can be observed by turning the tube containing the lysate by 180° (clot endpoint LAL) or, in a more quantitative way, by turbidimetric or chromogenic LAL, which measures quantitatively time of the clotting. The basic principle has been improved and modified in many ways. A sensitivity of 0.0005 ng/ml (0.5 pg/ml or 0.005 EU/ml) was described by the developers. The sensitivity of a usual lysate for the clotting reaction lies in a range of 0.03 EU/ml corresponding to 3 pg/ml.

The lysate is prepared by placing the crabs in restraining racks and inserting a needle through the muscular hinge between the cephalothorax and the abdominal region. Hemolymph is then drawn from the cardiac chamber into a container with anticoagulant. After collection, the amoebocytes are centrifuged and the supernatant is discarded. After 2-3 washing steps, the cells can be subjected to osmotic shock by adding pyrogen free distilled water and the intracellular lysate is released. Other methods exist, though. The product is then lyophilized and remains stable for ca. 3 years.

One of the drawbacks of the LAL is that it only detects endotoxin. Contaminations of drugs with Gram-positive bacteria or their fragments, e.g. with *Staphylococcus species* or spore forming bacteria like *Bacillus cereus* (a usual contaminant of manufacturing facilities in pharmaceutical industry), are not an unlikely event. Another one is that it does not distinguish between different biological potencies of LPS preparations, which can differ vastly in the whole blood of a mammal.

### Whole blood pyrogen test

A new way of measuring pyrogen has been introduced in 1995 by Hartung and Wendel. Basically, human blood is diluted in physiological saline and brought together with pyrogens or drugs suspected of being contaminated. In the case of pyrogenicity, the monocytes are challenged to produce cytokines *in vitro,* and the cytokines can be measured by specific ELISAs. The reaction is quantitative, that is, the more pyrogens are in the sample, the more cytokines are produced, and the test is performed in the relevant species, i.e. the reaction of man is tested. However, the tests available have a significant drawback. If only one cytokine is tested during monocyte activation it is possible to obtain a false-negative result when testing non-endotoxin pyrogens. Particular non-endotoxin pyrogens do not induce all pro-inflammatory cytokines.

EP 0 741 294 A2 discloses a method for analyzing substances with respect to pyrogenicity. A method is disclosed which makes use of interleukin-1. The method makes singular use of interleukin-6 and the method makes singular use of TNF.

It would be advantageous to have available an *in vitro* assay as well as substances that would enable pyrogen testing, make rabbit and other tests involving animals dispensable and, produce reliable results with respect to the presence of all possible pyrogens.

Further, it would be advantageous to have available a kit for performing said test which is easy to handle, gives a reliable result and is not expensive. The Limuls polyphemus test for example, has some inherent limitations. It can not detect any pyrogens except for endotoxin, and there is phylogenic distance between the horseshoe crab and higher vertebrates.

The novel pyrogen test should be based on *in vitro* reactions that are highly relevant to the above fever-inducing mechanisms in humans. The required test should be able to include test systems with human blood, isolated primary cells from human blood, and cell lines of the human monocyte/macrophage type. As an alternative to freshly drawn or prepared material, all of these monocyte sources can be used as cryo-preserved preparation.

The test system would need to be sensitive and would need to satisfy the practical demands, it would need to be simple and have low variability.

### Brief description of the invention

The invention relates to a method for detecting the presence of pyrogens in a sample comprising the steps of subjecting said sample to a solution comprising monocytes, incubating the sample and the solution, detecting in one reaction vessel the presence of at least two endogenous pyrogens. The invention further pertains to the use of the inventive method for evaluating the safety or possible contamination of a product for human or animal use. Further the invention relates to a kit for performing the inventive method, wherein the kit comprises two or more anti-endogenous-pyrogen-antibodies for detecting a pyrogen or at least two nucleic acid probes or primers for detecting a messenger RNA (mRNA) encoding an endogenous pyrogen. The invention further relates in particular to an ELISA kit, more in particular to an ELISA kit which comprises at least three anti-endogenous pyrogen antibodies selected from anti-IL-6, anti-IL-1, anti-TNF-α, anti-prostaglandin E₂ (PGE₂) and anti-neopterin.

### Detailed description of the invention

The invention relates to a method for detecting the presence of a pyrogen in a sample comprising the steps of (a) subjecting said sample to a solution comprising monocytes, (b) incubating the sample and the solution, and (c) detecting in one reaction vessel, the presence of at least two endogenous pyrogens in the solution of step (b). Thus, after incubation an aliquot or all of the solution from step (b) may be used for the detection step (c).

The inventors have astonishingly found that single detection of endogenous pyrogens often leads to false-negative results with respect to the presence of a pyrogen in a given sample. Further, the inventors have astonishingly found that two or more endogenous pyrogens may be detected in one reaction vessel most advantageously in a simultaneous manner.

Herein, "one reaction vessel" must not necessarily mean "one vessel". It may also be understood as "on one solid phase" or more generally in the context of "one simultaneous measurement". When making use of primers for RT-PCR the primers are all preferentially in one vessel. However, when making use of probes and hybridization techniques one vessel means one array or solid phase. In preferred embodiments where the invention relates to the use of, *e.g.* antibodies, said antibodies are actually present in one reaction vessel and/or one or more of them is attached to one solid phase, preferentially all are attached, whereas in such preferred embodiments where the detection is done making use of the messenger RNAs produced by the gene encoding the endogenous pyrogen, said mRNAs may be detected on one array. However, in a preferred embodiment said "one reaction" vessel is actually one reaction vessel. Most preferentially it is one reaction vessel and the antibodies used in preferred embodiments are attached to said vessel.

In a preferred embodiment the endogenous pyrogens are selected from the following substances, IL-1β, IL-6, TNF-α, prostaglandine E₂(PGE₂), and neopterine. Not only have the inventors astonishingly found, that when using prior art techniques that some exogenous pyrogens do not result in an endogenous pyrogen reaction but, more importantly, the inventors have found that monocytes from different origins give different endogenous pyrogen reactions when using prior art techniques. There was thus a great need for inventing a reliable system.

One of the strengths the method according to the invention has, is the great *in vitro*/*in vivo* correlation with respect to the presence of exogenous pyrogens. In fact, the method according to the invention makes it possible to calculate the amount of pyrogen which will ultimately, when given, *e.g.* intraveneously to a human individual, result in an elevation of body temperature. The fever threshold of humans is represented by 50 pg/ml (0.5 EU/ml) of a standard endotoxin in their peripheral blood. Exactly that value is mapped in the pyrogen test using human whole blood and has been chosen as the "cut off" of the test. In order to ensure this sensitivity, in every measurement an internal control consisting of an endotoxin spike (final concentration 50 pg/ml) is used.

In a particularly preferred embodiment at least the following three endogenous pyrogens are detected in one reaction vessel, IL-1β, IL-6 and TNF-α. Further pyrogens are optionally additionally detected.

The method according to the invention has a detecting step. The detecting step is preferentially done by applying a method selected from, (a) measuring and/or detecting the mRNA transcript of the genes encoding the endogenous pyrogens, (b) using an optical device such as a cytofluorometer for detecting the endogenous pyrogens, (c) detecting the protein of the endogenous pyrogens.

One skilled in the art will appreciate that there are a number of nucleic acid based methods for detecting mRNA transcripts from genes. It is important to apply a method which is able to detect the mRNA transcripts from the endogenous pyrogen genes. Such methods may be selected from the group of real-time-PCR (RT-PCR) microchip-array-detection as well as other methods available to one skilled in the art. In a preferred embodiment one skilled in the art will use an online quantitative RT-PCR method and/or a chip-based method. RT-PCR may be performed using a real-time PCR machine like the thermo-cycler (Roche Diagnostics, Mannheim, Germany). One skilled in the art will be able to design probes and/or primers for detecting said mRNA transcripts. The nucleic acid sequences of the genes encoding the endogenous pyrogens may be found in the respective databases such as the database containing the sequences of the human genome.

In one embodiment of the present method an optical device may be used such as a cytofluorometer for detecting the endogenous pyrogens (intracellular in the monocytes) of the present invention. Such an optical method in particular one using a cytofluorometer will or may encompass immunostaining. Immunostaining may then, *e.g.* be analyzed with an XR-4C cytofluorometer (Beckmann Coulter) or a FACS-Calibur (Becton Dickinson, BD). Another embodiment makes use of the so called Cytometric Bead Array provided by BD in which cytokines are fixed to antibody-coated, fluorescence labeled beads which trap the respective cytokine. These beads can be used as solid phase for more than one cytokine.

In a preferred embodiment the detection is done by detecting the endogenous pyrogen protein.

In a particularly preferred embodiment said detecting the protein is done in an ELISA format using anti-endogenous-pyrogen-antibodies against said endogenous pyrogens. In a further preferred embodiment at least one of the anti-endogenous-pyrogen-antibodies is attached to a solid phase. In a particularly preferred embodiment two or more of the anti-endogenous pyrogen-antibodies is attached to a solid phase.

In one embodiment of the invention the amount of one or more of the anti-endogenous-pyrogen-antibodies is varied from, all being present in equal molarities to all being present in different molarities, depending on the affinity of the antibodies for the endogenous pyrogens. The inventors have found that the anti-endogenous-pyrogen-antibodies of the present invention may have varying affinities for the endogenous pyrogens. In order to cope with this problem the molarities of the anti-endogenous-pyrogen-antibodies is adjusted accordingly. Thus, in the event that a particular anti-endogenous-pyrogen-antibody has a low affinity for its endogenous pyrogen the molarity of said anti-endogenous-pyrogen antibody may be higher within said one reaction vessel, in particular higher than the molarity of an anti-endogenous-pyrogen-antibody with a higher affinity for its endogenous pyrogen.

The solution according to the invention comprising the monocytes is selected from the group of monocytes from apheresis, mononuclear cells, whole blood, preserved blood, frozen blood and blood fractions. According to the invention, it is important that the solution according to the invention comprises monocytes. Monocytes belongs to the leukocytes (white cells) of the human body's immune system that protect against pathogens by moving quickly to sites of infection in the tissues. Monocytes are 13 to 25 µm in diameter. Monocytes are typically identified by flow-cytometry using surface expression of the protein CD14, a receptor for bacterial endotoxin. Monocytes are produced in the bone marrow from hematopoietic stem cell precursors, circulate in the blood stream for about 1 to 3 days and then typically move into tissue through the body. Monocytes, which migrate from the blood stream to other tissues are called macrophages.

Encompassed by the invention and falling within the definition herein are also the peripheral blood mononuclear cells (PBMCs). They are blood cell having a round nucleus such as a lymphocyte or a monocyte which gave the name for the blood cell fraction. These blood cells are critical components in the human immune system to fight towards infections and adapt to intruders. Peripheral blood cells are the cellular components of blood consisting of red blood cells, white blood cells and platelets, which are found within the circulating pool of blood and not sequestered within the lymphatic system, spleen, liver or bone marrow. Thus, according to the definition herein, a monocyte according to definition is a cell in a body fluid which is able to express CD 14.

In a preferred embodiment the monocyte solution according to the invention is whole blood. It is easier to use for pyrogen-detecting purposes human or animal whole blood such as, *e.g.* freshly obtained blood from human healthy donors, without separation of single components or cells. For animal pyrogen delection one skilled in the art will provide for species specific or animal specific anti-endogenous-pyrogen-antibodies. In this system leukocytes are in their natural composition and environment in all serum components are present that can influence the actions of exogenous pyrogens. A special advantage is that components can be added to whole blood that retard or prevent blood clotting, such as, *e.g.* citrate, in a final concentration of 0,38% or heparine (sodium heparinate) that can be used during the incubation without effecting or falsifying the reaction described for the invention. Using heparine, the complement system is potentially involved in a monocyte activation as some pyrogens (*e.g.* immune complexes) can activate the complement cascade. The letter leads to the release of the complement factors C3a and C5a, the so called anaphylactins, also able to activate monocytes. In contrast, anticoagulation of the blood by citrate will lead to the absence of Ca⁺⁺ and Mg⁺⁺ in the pyrogen test because both are bound by citrate. Divalent cations are needed for the complement activation as well as for inhibition of the coagulation cascade. It has been found to be useful to dilute the whole blood, for instance, to 8% of the original concentration with cell culture medium or with physiological sodium chloride solution. This is done in particular when the method of detecting according to the invention is an ELISA. It is needless to say that any technical devices used, such as laboratory equipment or compounds to be added including anti-coagulants or dilutives, has to be free of exogenous pyrogens. During the incubation according to the method which will be outlined in more detail below, where preventatively antibiotics such as Penicillin, Streptomycin, Chloramphenicol, Amphotericin or combinations of them can be present, a relevant contamination has to be excluded.

In a preferred embodiment according to the method of the present invention the whole blood of the present invention comprises blood from multiple donors in a preferred embodiment from at least three donors, more preferred from at least six donors, most preferred from at least ten donors. Since there is an individual susceptibility in humans towards some non-endotoxin pyrogens, pooling of blood from different donors will increase the probability of their detection substantially.

In one embodiment of the invention, the invention relates to the use of the method according to the invention for evaluating safety of possible contamination of a product for use with humans or animals. Such a use may be, *e.g.* the testing of pharmaceuticals, such as routine testing of blood products or infusion products.

The invention also relates to a kit for performing the method according to the invention, wherein the kit is able to detect two ore more endogenous-pyrogens for detecting the protein of the endogenous pyrogens or at least two nucleic acid probes or primers for detecting the mRNA of said genes encoding the endogenous pyrogens.

The invention also relates to a kit, wherein the kit is an ELISA kit and the kit is able to detect at least three enodgenous pyrogens selected from, IL-6, IL-1β, TNF-α, Prostglandin E₂(PGE₂), and Neopterine.

In a particularly preferred embodiment, the kit according to the invention is an ELISA kit for detection of IL-6, IL-1β and TNF-α. In a preferred embodiment it contains two, in a particularly preferred embodiment it contains three of the antibodies selected from anti-IL-6, anti-IL-1β and anti-TNF-α in one vessel.

In a preferred embodiment, the antibodies are present in equal or varying molar ratios.

### Examples

### Example 1:

Anti-bodies (anti-IL-1β, anti-IL-6, anti-TNF- α) are bound to the wells of an ELISA-plate. This way, in one reaction all three cytokines may be measured. This great advantage herein lies in that also unusual non-endotoxin pyrogens are safely found. According to the invention, the antibodies may be bound directly or an ELISA-plate is used, which has a bound antimouse-IgG antibody ("Even Coat"), R&D, USA. Commercially available ELISA-plates are incubated with a mixture of commercially available anti-IL-1β, anti-IL-6, and anti-TNF-α-antibodies (R&D, USA), making use of an advantageous reaction mixture, these are then washed and blocked with commercially available reagents. Said plates may be stored in dried or frozen status or may be used directly for pyrogen testing. For this purpose the solution comprising the monocytes which was subjected to the sample to be tested for the presence of the pyrogen is (this was done in an incubation over night in a CO₂-incubator) is given directly into the ELISA-plate. There is no need to discard debris or cells or supernatants. The ELISA-plates are incubated at room temperature and washed with a commercially available washer. Thereafter, commercially available biotin-labeled anti-IL-1β, anti-IL-6, and anti-TNF-antibodies (R&D, USA) are incubated and thereupon the plates are washed. After this step, a streptavidin-peroxidase conjugate is added and a further incubation and washing step performed. The ELISA is then developed using a standard peroxidase-chromogen. The plates are measured in a commercial plate reader. For calibration a positive control the ELISA optionally contains a mixture/combination of standards of the three cytokines (this may be done in a dilution series) for all steps of the method according to the invention.

### Materials

A) The following plates may be used for the method: Nunc F96 Maxisorp, product-No. 442404
B) DuoSet ELISA Development Kits of R&D:
   IL-1β (Catalog-No. DY201)
   IL-6 (Catalog-No. DY206)
   TNF-α (Catalog-No. DY210)
C) PBS o. Ca and Mg and washing buffer (containing additionally 0.05% Tween 20)
D) Reagent Diluent, made of PBS + 1% BSA; filtered sterile with a a 0.2 µm-filter
E) Substrate = TMB: either Substrate Reagent Pack of R&D (Catalog-No. DY999) or TMB Peroxidase EIA substrate Kit of Biorad (Catalog-No. 172-1066)
F) 1N (or also 2N) H₂S0₄ used as stop solution

### Procedure: all materials see section "Materials" above

1 .Coating with Capture Antibody: 1 part IL- 1β + 1 part IL-6 + 2 parts TNF-α

| Plate No. | IL-1β µl | +IL-6 µl | +TNF µl | + PBS (without additives) ml |
|---|---|---|---|---|
| 1 | 58 µl | 58 µl | 116 µl | 10,2 ml |
| 2 | 116 µl | 116 µl | 232 µl | 20,4 ml |
| 3 | 174 µl | 174 µl | 348 µl | 30,6 ml |
| 4 | 232 µl | 232 µl | 464 µl | 40,8 ml |
| 5 | 290 µl | 290 µl | 580 µl | 51,0 ml |
| 6 | 348 µl | 348 µl | 696 µl | 61,2 ml |
| 7 | 406 µl | 406 µl | 812 µl | 71,4 ml |
| 8 | 464 µl | 464 µl | 928 µl | 81,6 ml |
| 9 | 522 µl | 522 µl | 1044 µl | 91,8 ml |

100 µl/well are plated, plates are closed and incubated over night at room temperature.
2. Plates are washed three times with washing buffer (Programm PYRO_1 Tecan plate washer)
3. Blocking solution (PBS + 10% FCS + 2,5 % NaN₃) 2 h at room temperature, 100 µl/well
4. Washing - thereafter plates are sealed and stored at -20°C or subjected to the following procedure
5. Mixes are prepared for the following standard rows, a two step procedure is used:
a) pre-dilution of the three cytokines (end concentration each 3000 pg/ml)

| Plate No. | IL-1 | +IL-6 | +TNF | +Reagent Diluent |
|---|---|---|---|---|
| 1-5 | 6 µl | | | 215 µl |
| | | 10 µl | | 220 µl |
| | | | 3 µl | 288 µl |
| 6-10 | 12 µl | | | 430 µl |
| | | 20 µl | | 440 µl |
| | | | 6µl | 576 µl |

b) working dilution of the cytokine mixture:
for 1-5 plates pipet 200 µl of the three pre-dilutions = total volume of 600 µl (end concentration of each cytokine is now 1000 pg/ml), *i.e.* for plates 6-10 each 400 µl of the pre-dilutions solution are pipetted together = total volume of 1200 µl (final concentration of each cytokine is now 1000 pg/ml).
Thereupon, a second dilution series of the cytokine mixture is performed that means the same parts of the cytokine mixtures are mixed with same parts of the reagent diluent, i.e. 300 µl cytokine mixture + 300 µl ReagentDiluent for plates 1-5 (600 µl+600 µl for plates 6-10). This results in cytokine mixture concentrations of 1000/500/250/125/65,5/31,25/15,63 pg/ml; the negative control is performed with Reagent Diluent solely..
6. This standard series including negative controls and the probes which were mixed well beforehand are applied to the microtiter plates (50 µl/well). The plates are sealed and incubated for 1 h at room temperature on the shaker.
7. Washing
8. Mixing preparation of detection antibodies:

| Plate No. | IL-1β µl | +IL-6 µl | +TNF µl | + Reagent Diluent ml |
|---|---|---|---|---|
| 1 | 58 µl | 58 µl | 116 µl | 10,2 ml |
| 2 | 116 | 116 | 232 | 20,4 |
| 3 | 174 | 174 | 348 | 30,6 |
| 4 | 232 | 232 | 464 | 40,8 |
| 5 | 290 | 290 | 580 | 51,0 |
| 6 | 348 | 348 | 696 | 61,2 |
| 7 | 406 | 406 | 812 | 71,4 |
| 8 | 464 | 464 | 928 | 81,6 |
| 9 | 522 | 522 | 1044 | 91,8 |

100 µl/well is incubated for 1 h at room temperature on the shaker
9. Washing
10. Preparation of the Streptavidin-HRP working dilution (see details on the bottle), here 1:200, *i.e.:*

| Plate No. | HRP µl | + Reagent Diluent ml |
|---|---|---|
| 1 | 53 µl | 10,6 ml |
| 2 | 106 | 21,1 |
| 3 | 159 | 31,6 |
| 4 | 212 | 42,2 |
| 5 | 256 | 52,7 |
| 6 | 318 | 63,3 |
| 7 | 371 | 73,8 |
| 8 | 424 | 84,4 |
| 9 | 477 | 94,9 |

Add 100 µl/well incubate for 20 minutes in a dark environment.
11. Washing.
12. Substrate incubation with TMB: per plate either 5 ml (half the content) from bottle A and B (Substrate Reagent Pack , R&D) or 9 ml bottle A + 1 ml bottle B (TMB Peroxidase EIA substrate Kit, Biorad) are mixed prior to use; 100 µl/well are used; the solution is to be protected from light.
13. After no more than 15 minute the reaction is stopped with 50 µl/well 1NH₂SO₄ and thereupon the plate is briefly shaken.
14. Photometric measurement at 450 nm (reference wavelength 540 nm) is to be performed as soon as possible.

### Example 2:

Principle of alternative pyrogen testing
(In vitro Pyrogen Test, Monocyte Activation Test, Monocyte Cytokine Release Test, Monocyte pro-inflammatory cytokine release test etc.)
Part 1:
   Incubation of cell culture with the sample (to be tested for pyrogens)
   Monocyte sources according to the invention; detailed list:
   Diluted human whole blood, freshly drawn
   Diluted human whole blood, cryo-preserved
   Isolated Peripheral Blood Mononuclear Cells (PBMC)
   isolated by gradient zentrifugation of whole blood,
   gradient has to be pyrogen-free!
   furthermore: gentle handling for prevention of monocyte activation obligatory
   Isolated Peripheral Blood Mononuclear Cells (PBMC), cryo-preserved/deep frozen
   Isolated human monocytes, e.g. obtained by Monocyte-Apheresis in blood transfusion facility
      (resp. strong enrichment of monocytes, they do contain other blood cells)
   Isolated human monocytes, obtained by any other procedure
   Isolated human monocytes, cryo-preserved/deep frozen
      (primary cells all treated with anti-coagulants)
   Cell lines of human monocytes/ monocytic cell lines (e.g. THP-1, MonoMac-6)
   Pooling of monocytes derived from different donors
   In order to improve the probability to detect non-endotoxin pyrogens monocytes from different donors can be pooled.This can be done with whole blood (fresh or frozen), PBMC (fresh or frozen), isolated monocytes (fresh or frozen)
   Control reagent: typically endotoxin/lipopolysaccharide, others possible (e.g. non-endotoxin-pyrogens)
   Example (current routine in PEI)
   The cryopreserved heparinized blood (final blood dilution with cryoprotective solution 1:2) is thawed in a defined procedure. Afterwards the blood is diluted 1:5 with RPMI 1640 (low-endotoxin grade) to a final blood dilution of 1:10. The sample is added per reaction vessel to achieve a sample dilution of 1:11 (1 part sample, 10 parts diluted blood, works also in other correlations, e.g. 1:6 etc.)
   Incubation overnight in an CO₂ incubator at 37°C.
Part 2: ELISA
   Thereafter, mixing of the incubated suspension (no separation between supernatant and cells resp. debris necessary!) and pipetting into ELISA-plates (96 well), different volumes possible, e.g. 50 µl, 100 µl, 200 µl per well, in our case Tri-Zytokine-ELISA-Plates
   Incubation of plates at room temperature (or higher) for e.g. 1 hour
   Washing of Plates
   Incubation with conjugates, in our case with a mixture of Anti-IL-1β, Anti-IL-6, Anti-TNF□ at room temperature (or higher) for e.g. 1 hour
   Washing
   Incubation with Streptavidine-Peroxidase-Conjugate at room temperature (or higher) for e.g. 1 hour
   (theoretically Anti-Zytokine-Antibodies and Streptavidine-Peroxidase-Conjugate can be mixed and incubated together in 1 step)
   Washing
   Development of ELISA with a chromogenic substrate for peroxidase

Control reagent: Mixture of standards of the three cytokines

### Figures

Figures 1a to 1d
   Pooled fresh blood of 4 donors was directly inoculated separately with different bacterial species capable of growing in blood (1000, 100, 10, 1, 0 colony forming units (cfu/ml) or incubated in the presence of human platelets (samples 1-5) contaminated with different bacterial species capable of growing in blood. In some cases (table *Staphylococcus epidermidis,* table *Staphylococcus aureus)* the release of single cytokines (e.g IL-1β and IL-6) is strongly reduced up to abolished in comparison to the other cytokines detected.
Figure 2
   Detection of either IL-1β, IL-6 or TNF alphasolely (1000 pg/ml- 0 pg/ml) or the mix of IL-1β, IL-6 and TNF alpha (ratio 1 + 1+ 1) in a "Three-Cytokine-ELISA" (Plate coated wit a mix of anti-human I1-1β, anti-human IL-6 and anti-human TNF alpha.
Figure 3a to 3d
   Fresh blood and cryopreserved blood (-80°C) of 4 identical donors (both single blood and pooled blood) was incubated for 16h in the presence of a LPS-dilution series (2500 pg/ml to 0 pg/ml). ELISA was performed either on single cytokine ELISA or a Tri-cytokine ELISA.

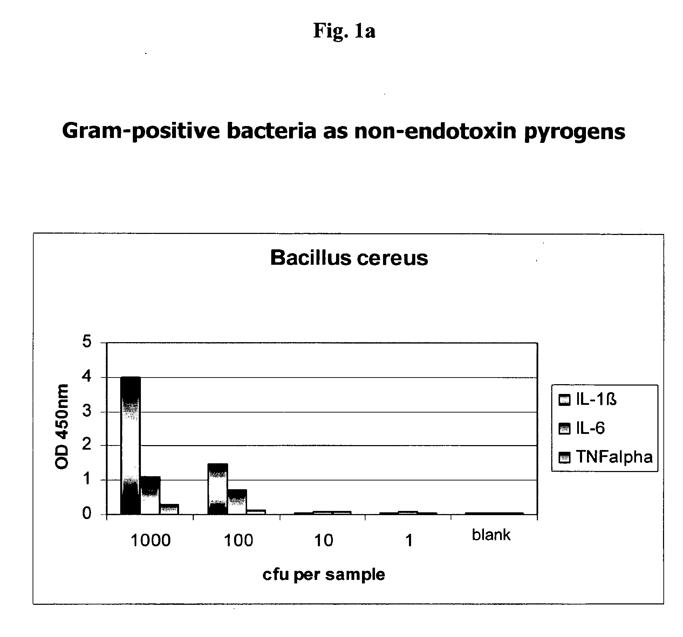

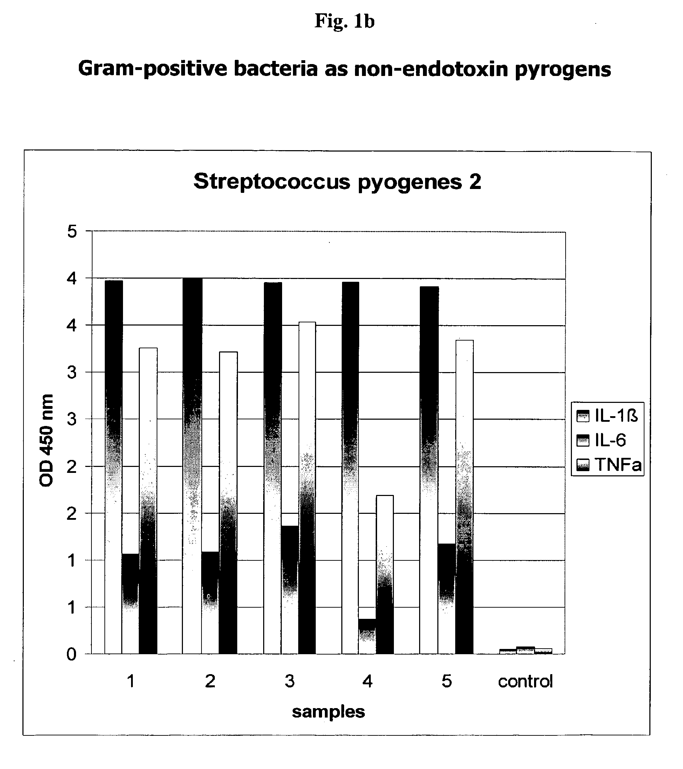

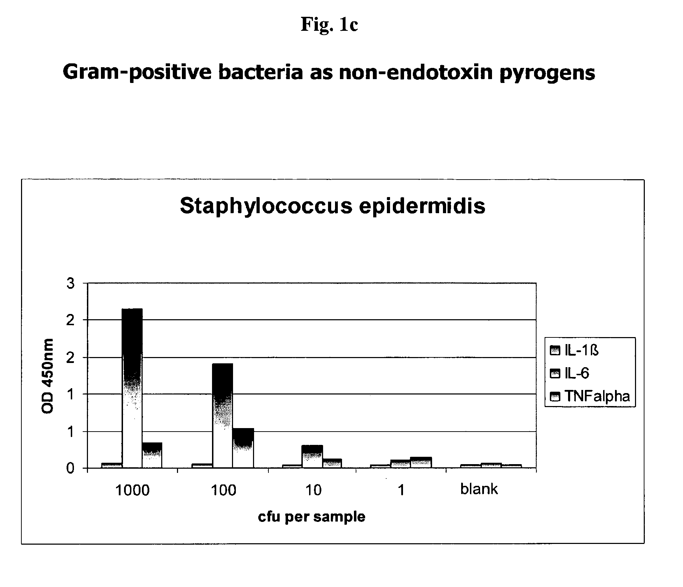

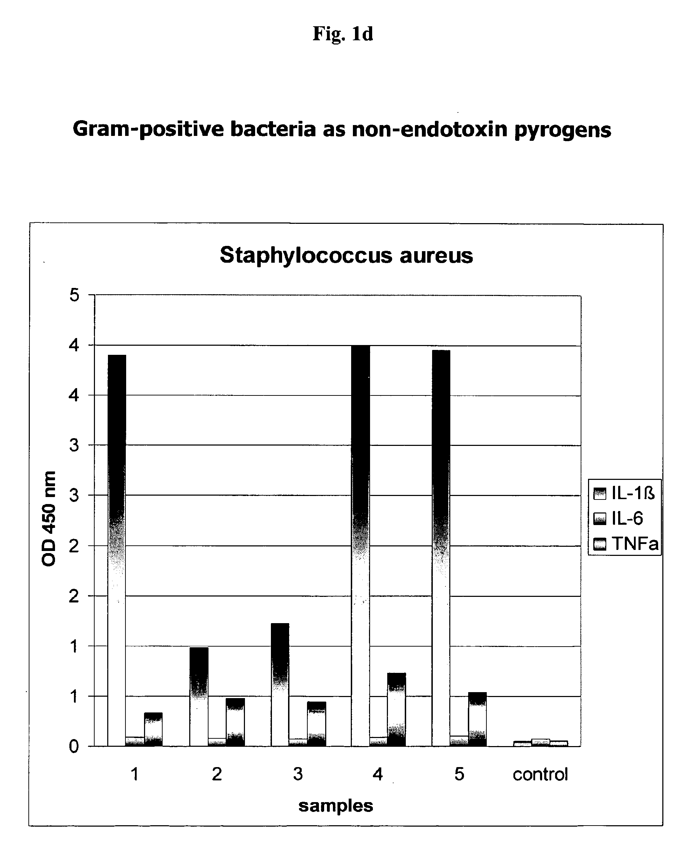

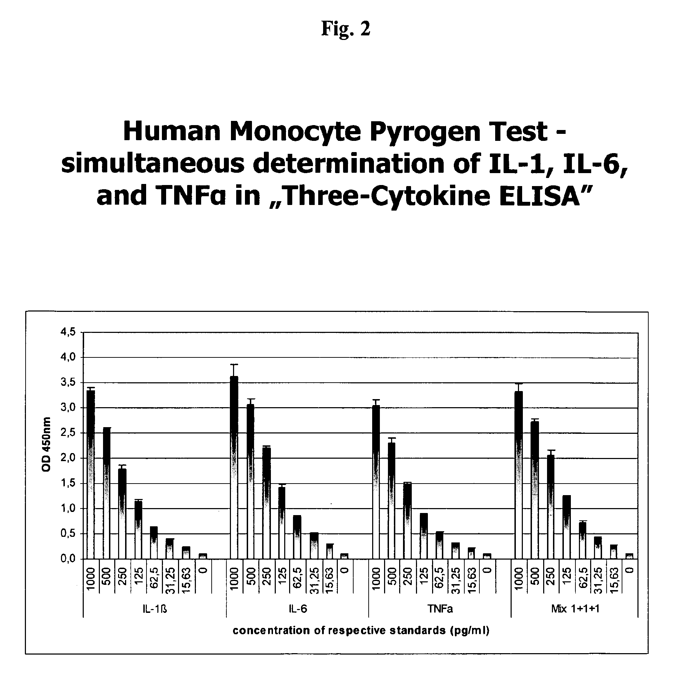

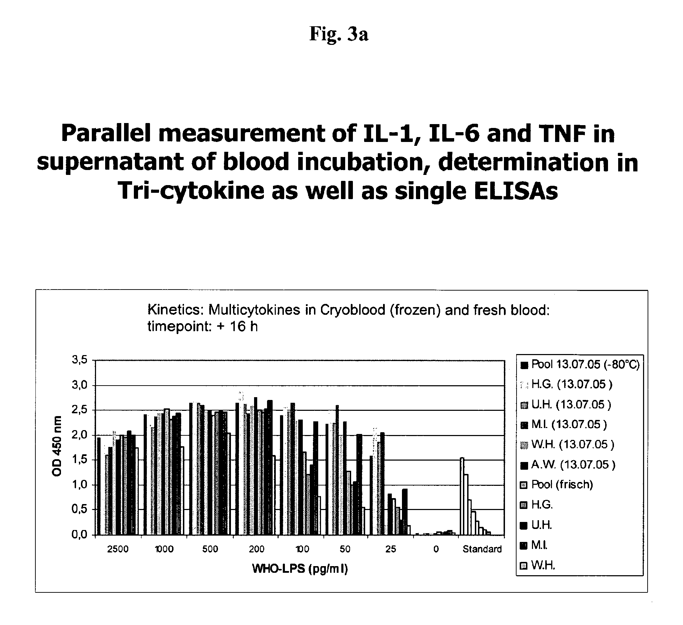

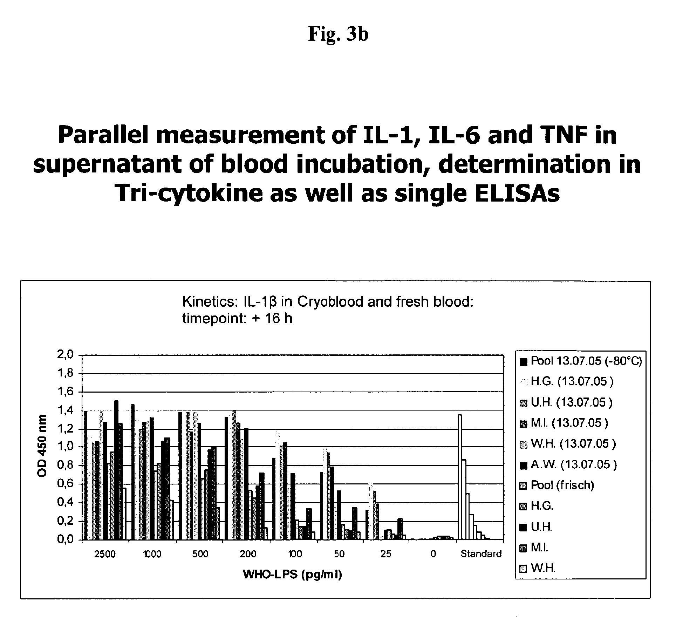

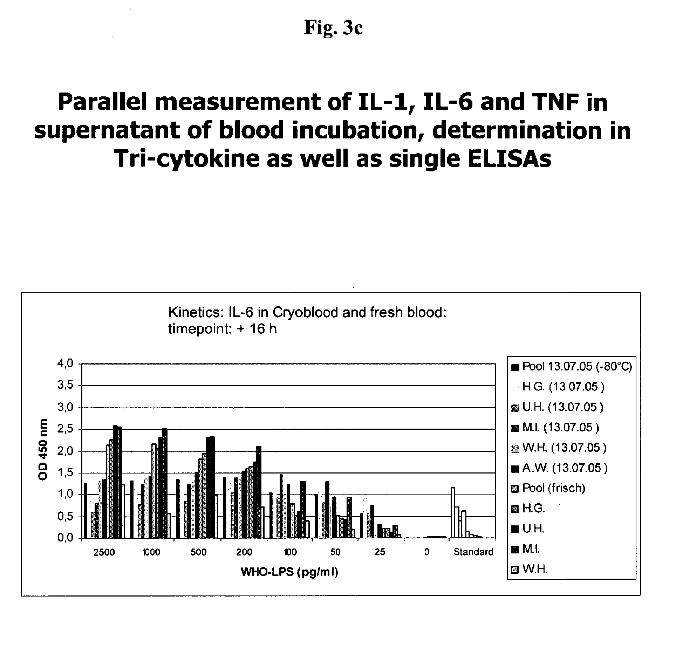

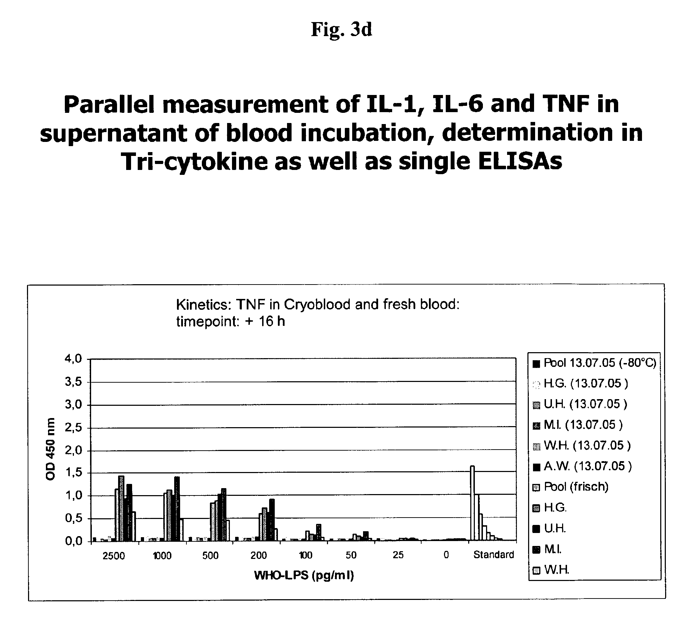

## Claims

1. A method for detecting the presence of a pyrogen in a sample comprising the steps of:
a) subjecting said sample to a solution comprising monocytes,
b) incubating the sample and the solution and
c) detecting, in one reaction vessel, the presence of at least two endogenous pyrogens in the solution from step b).

2. Method according to claim 1, wherein the endogenous pyrogens are selected from the following substances, IL-1β, IL-6, TNF-α, prostaglandin E2 (PGE2) and neopterin.

3. Method according to claim 2 wherein, the following three endogenous pyrogens are at least detected in one reaction vessel, IL-1β, IL-6 and TNF-α.

4. Method according to claims 1 to 3, wherein,
said detecting is done by applying a method selected from,
a) measuring and/or detecting the mRNA transcript of the genes encoding the endogenous pyrogens,
b) using an optical device, such as a cytofluorometer for detecting the endogenous pyrogens and
c) detecting the endogenous pyrogen protein.

5. Method according to claim 4, wherein said detecting the protein is done in an ELISA format making use of antibodies against said endogenous pyrogen protein.

6. Method according to claim 4 or 5 wherein, at least one of the anti-endogenous-pyrogen-antibodies is attached to a solid phase.

7. Method according to claims 5 or 6 wherein, the amount of one or more of the anti-endogenous-pyrogen-antibodies is varied from, all being present in equal molarities to all being present in different molarities, depending on the affinity of the antibodies for the endogenous pyrogens.

8. Method according to any of the above claims, wherein the solution comprising monocytes is selected from apheresis monocytes, peripheral blood mononuclear cells, whole blood, preserved blood, frozen blood and blood fractions.

9. Use of a method according to claims 1 to 6 for evaluating safety or possible contamination of a product for use with humans or animals.

10. Kit for performing the method according to claims 1 to 8, wherein the kit comprises:
a) two or more anti-endogenous-pyrogen-antibodies for detecting the endogenous pyrogen protein or,
b) at least two nucleic acid probes or primers for detecting the mRNA of said genes encoding the endogenous pyrogens.

11. Kit according to claim 10 for performing the method according to claims 1 to 8, wherein, the kit is an ELISA kit and the kit enables the detection of three endogenous pyrogen protein selected from, IL6, IL1, TNF-α, prostaglandin E2 (PGE2) and neopterin.

12. Kit according to claim 11, wherein the kit is an ELISA kit and comprises at least three antibodies selected from anti-IL-6, anti-IL-1, anti-TNF-α, anti-prostaglandin E2 (PGE2) and anti-neopterin.

13. Kit according to claim 11, wherein the ELISA kit comprises anti-IL6, anti-IL1 and anti-TNF-α bound to a solid phase, preferentially a micro-titer plate.

14. Kit according to claims 11 or 12, wherein the antibodies are present in differenty molar ratios corresponding to their affinity for their respective endogenous pyrogen protein.

15. Kit according to claim 14, wherein an antibody with a low affinity for its endogenous pyrogen protein is present at a higher molar concentration than an antibody with a high affinity for its endogenous pyrogen protein.
